(19) Europäisches Patentamt
European Patent Office
Office européen des brevets

(11) Numéro de publication : **0 374 041 B1**

(12) **FASCICULE DE BREVET EUROPEEN**

(45) Date de publication du fascicule du brevet : **17.06.92 Bulletin 92/25**

(51) Int. Cl.$^5$ : **C07D 277/82,** A61K 31/425

(21) Numéro de dépôt : **89403460.2**

(22) Date de dépôt : **13.12.89**

(54) **Médicaments à base de dérivés de la benzothiazolamine-2, dérivés et leur préparation.**

Le dossier contient des informations techniques présentées postérieurement au dépôt de la demande et ne figurant pas dans leprésent fascicule.

(30) Priorité : **15.12.88 FR 8816548**
**13.07.89 FR 8909484**

(43) Date de publication de la demande : **20.06.90 Bulletin 90/25**

(45) Mention de la délivrance du brevet : **17.06.92 Bulletin 92/25**

(84) Etats contractants désignés : **AT BE CH DE ES FR GB GR IT LI LU NL SE**

(56) Documents cités :
**EP-A- 0 050 551**
**EP-A- 0 282 971**
**FR-A- 2 357 553**
**CHEMICAL ABSTRACTS, vol. 109, no. 5, 1 août 1988, page 57, résumé no. 31973t, Columbus, Ohio, US; J.M. STUTZMANN et al.:"Riluzole, a glutamate antagonist, enhances slow wave and REM sleep in rats"**
**CHEMICAL ABSTRACTS, vol. 47, no. 10, 25 mai 1953, page 4771a-f, Columbus, Ohio, US; L.M. YAGUPOL'SKII et al.: "Cyanine dyesfrom derivates of 6-(trifluoromethylthio)benzothiazole"**
**J.M. Stutzmann, NEUROSCI. LETT., vol. 88, no. 2, pages 195-200, 1988**

(73) Titulaire : **RHONE-POULENC SANTE**
**20, avenue Raymond Aron**
**F-92160 Antony (FR)**

(72) Inventeur : **Audiau, François**
**9 rue Guérin**
**F-94220 Charenton Le Pont (FR)**
Inventeur : **James, Claude**
**31bis Avenue Gambetta**
**F-75020 Paris (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 374 041 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne des médicaments contenant en tant que principe actif au moins un composé de formule :

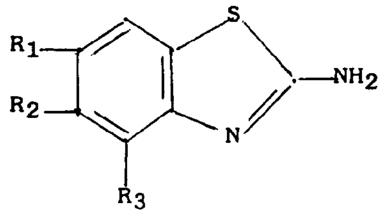

(I)

ou un sel d'un tel composé avec un acide minéral ou organique, les nouveaux composés de formule (I) et leurs procédés de préparation.

Le brevet EP 50551 et l'article paru dans Neuroscience letters 82(2), 195(1988) décrivent l'amino-2 trifluorométhoxy-6 benzothiazole comme anticonvulsivant, anxiolytique, hypnotique et comme antiglutamate.

Ce composé et des dérivés de l'amino-2 benzothiazole sont également décrits dans la demande de brevet EP 282971 comme agents cérébrovasculaires.

Le butyl-6 amino-2 benzothiazole est décrit dans le brevet FR 23575553. La trifluorométhylthio-6 benzothiazolamine-2 est décrite dans Zh. Obshch. Khim., 22, 2216 (1952) (Chem. Abst. 47, 4771 c) et 33(7), 2301 (1963). Aucune propriété pharmacologique n'est mentionnée pour ces deux composés.

Les composés de formule (I) ou leurs sels incorporés en tant que principe actif dans les médicaments selon l'invention sont ceux pour lesquels :

– soit $R_1$ représente un radical polyfluoroalcoxy, trifluoro-2,2,2 éthyle, pentafluoroéthyle, tertbutyle, triméthylsilyle ou trifluorométhylthio et $R_2$ et $R_3$ représentent un atome d'hydrogéne,

– soit $R_1$ représente un radical polyfluoroalcoxy, $R_2$ représente un atome d'hydrogéne et $R_3$ représente un radical alkyle, amino ou phénylalkyle.

– soit $R_1$ représente un radical polyfluoroalcoxy, $R_2$ représente un radical amino et $R_3$ représente un atome d'hydrogéne à l'exception de la trifluorométhoxy-6 benzothiazolamine-2.

Les radicaux polyfluoroalcoxy préférés sont les radicaux pentafluoroéthoxy, trifluoro-2,2,2 éthoxy, tétrafluoro-1,1,2,2 éthoxy, trifluorométhoxy et pentafluoro-2,2,3,3,3 propoxy.

Dans les définitions qui précédent et celles qui seront citées ci-après, sauf mention contraire les portions alkyle et alcoxy ou les radicaux alkyle et alcoxy contiennent 1 à 4 atomes de carbone en chaîne droite ou ramifiée.

Les composés de formule (I), à l'exception des trifluorométhylthio-6 et trifluorométhoxy-6 benzothiazolamine-2, sont nouveaux et font partie en tant que tels de l'invention.

Les composés de formule (I) pour lesquels

– soit $R_1$ représente un radical polyfluoroalcoxy, tertbutyle, trifluoro-2,2,2 éthyle, pentafluoroéthyle et $R_2$ et $R_3$ représentent un atome d'hydrogène,

– soit $R_1$ représente un radical polyfluoroalcoxy, $R_2$ représente un atome d'hydrogéne et $R_3$ représente un radical alkyle, ou phénylalkyle peuvent être obtenus par action de brome et d'un thiocyanate de métal alcalin sur une amine de formule :

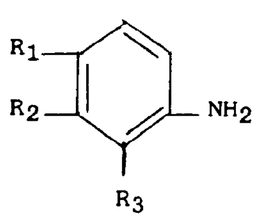

(II)

dans laquelle $R_1$, $R_2$ et $R_3$ ont les mêmes significations que précédemment.

Cette réaction s'effectue, généralement, au sein d'un solvant organique tel que l'acide acétique, à une température voisine de 20°C. Comme thiocyanate de métal alcalin, on utilise de préférence le thiocyanate de potassium.

Les amines de formule (II) peuvent être préparées par application ou adaptation des méthodes décrites

dans J. Org. Chem., 29, 1 (1964) ; Beilstein 12, 1166, dans les brevets US 3 920 444, US 2 436 100, DE 3 195 926, DE 2 606 982, EP 205 821 et dans les exemples.

Le composé de formule (I) pour lequel $R_1$ représente un radical triméthylsilyle et $R_2$ et $R_3$ représentent un atome d'hydrogéne, peut être obtenu par action de chlorotriméthylsilane sur le dérivé lithié en 6 de la N,N-bistriméthylsilyl benzothiazolamine-2 obtenu par action du butyllithium et de chlorotriméthylsilane sur la bromo-6 benzothiazolamine-2 puis hydrolyse du groupement N, N-bis-triméthylsilyle.

Ces réactions s'effectuent sans séparation du dérivé lithié dans un solvant inerte tel que l'hexane, le tétrahydrofuranne ou un mélange de ces solvants, à une température comprise entre -70°C et la température d'ébullition du milieu.

La bromo-6 benzothiazolamine-2 peut être préparée par application de la méthode décrite dans Beilstein 27, 184.

Les composés de formule (I) pour lesquels $R_1$ représente un radical polyfluoroalcoxy et soit $R_2$ représente un radical amino et $R_3$ représente un atome d'hydrogène, soit $R_2$ représente un atome d'hydrogéne et $R_3$ représente un radical amino, peuvent être obtenus par réduction d'un dérivé de formule :

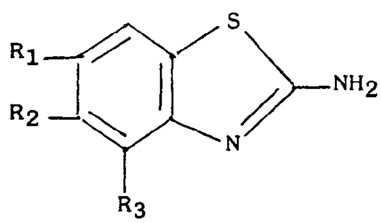

(III)

dans laquelle $R_1$ représente un radical polyfluoroalcoxy et soit $R_2$ représente un radical nitro et $R_3$ représente un atome d'hydrogéne, soit $R_2$ représente un atome d'hydrogène et $R_3$ représente un radical nitro.

Cette réduction s'effectue, généralement, au moyen de fer et d'acide chlorhydrique, au sein d'un alcool tel que l'éthanol ou le méthanol, à la température d'ébullition du solvant.

Les composés de formule (III) peuvent être obtenus par nitration de la polyfluoroalcoxy-6 benzothiazolamine-2 correspondante et séparation des deux produits.

Cette nitration s'effectue, généralement, au moyen d'un mélange sulfonitrique, à une température voisine de 0°C.

Les polyfluoroalcoxy-6 benzothiazolamine-2 peuvent être obtenues par application ou adaptation de la méthode décrite dans Zh. Obshch. Khim. 33, 2301 (1963).

Les mélanges réactionnels obtenus par les divers procédés décrits précédemment sont traités suivant des méthodes classiques physiques ou chimiques (évaporation, extraction, distillation, cristallisation, chromatographie, formation de sels).

Les composés de formule (I) sous forme de base libre peuvent être, éventuellement, transformés en sels d'addition avec un acide minéral ou organique, par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle décrite par I.P. LAPIN, J. Neural. Transmission, vol.54, 229-238 (1982) ; l'injection du glutamate par voie intracérébroventriculaire étant effectuée selon une technique inspirée de celle de R. CHERMAT et P. SIMON, J. Pharmacol. (Paris), vol.6, 489-492 (1975). Leur $DE_{50}$ est inférieure ou égale à 10 mg/kg.

Les composés de formule (I) présentent une toxicité faible. Leur $DL_{50}$ est généralement supérieure à 60 mg/kg par voie I.P. chez la souris.

D'un intérêt particulier sont les composés suivants :

– pentafluoroéthoxy-6 benzothiazolamine-2,
– tert-butyl-6 benzothiazolamine-2,
– trifluorométhoxy-6 benzothiazolediamine-2,5,
– trifluorométhoxy-6 benzothiazolediamine-2,4.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables peuvent être cités les sels d'addition avec les acides minéraux ou organiques tels que chlorhydrate, sulfate, nitrate, phosphate, acétate, propionate, succinate, benzoate, fumarate, maléate, oxalate, méthanesulfonate, iséthionate, théophyllineacétate, salicylate, phénolphtalinate et méthyléne-bis-β-oxynaphtoate.

Les exemples suivants montrent comment l'invention peut être mise en pratique.

## EXEMPLE 1

A une solution de 4,8 g de pentafluoroéthoxy-4 aniline dans 35 cm3 d'acide acétique on ajoute, sous balayage d'argon, 8,15 g de thiocyanate de potassium et on agite pendant 10 minutes à une température voisine de 20°C. A la solution ainsi obtenue, on verse goutte à goutte, en 35 minutes, une solution de 1,1 cm3 de brome dans 10 cm3 d'acide acétique à une température comprise entre 22 et 42°C ; on agite ensuite pendant 20 heures à une température voisine de 20°C. Le mélange réactionnel est versé sur un mélange d'eau et de glace (250 cm3), alcalinisé avec 50 cm3 d'ammoniaque à 28 % et extrait 2 fois avec 250 cm3 d'acétate d'éthyle au total. Après décantation, la solution organique est lavée à l'eau distillée jusqu'à pH 8, séchée sur sulfate de magnésium, filtrée et évaporée à 50°C sous pression réduite (20 mm de mercure ; 2,7 kPa). Le produit obtenu (6,3 g) est purifié par chromatographie sur colonne de silice (650 g, granulométrie : 0,063-0,200 mm) avec comme éluant un mélange de cyclohexane et d'acétate d'éthyle (50-50 en volumes) et recristallisé dans 400 cm3 de cyclohexane bouillant. On obtient 3,25 g de pentafluoroéthoxy- 6 benzothiazolamine-2 fondant à 156°C.

La pentafluoroéthoxy-4 aniline peut être préparée selon la méthode décrite par W.A. SHEPPARD, J. Org. Chem., 29, 1 (1964).

## EXEMPLE 2

On opère comme à l'exemple 1, à partir de 14,9 g de tert-butyl-4 aniline, 38,8 g de thiocyanate de potassium et de 5,1 cm3 de brome dans 150 cm3 d'acide acétique. Après purification sur colonne de silice (700 g, granulométrie : 0,063-0,200 mm) avec comme éluant un mélange cyclohexane-acétate d'éthyle (40-60 en volumes) et recristallisation dans 450 cm3 de cyclohexane bouillant, on obtient 12,2 g de tert-butyl-6 benzothiazolamine-2 fondant à 146°C.

La tert-butyl-4 aniline peut être préparée selon la méthode décrite dans le BEILSTEIN 12, 1166.

## EXEMPLE 3

On opère comme à l'exemple 1, à partir de (trifluoro-2,2,2 éthoxy)-4 aniline, de thiocyanate de potassium et de brome dans l'acide acétique pour obtenir la (trifluoro-2,2,2 éthoxy)-6 benzothiazolamine-2 qui fond à 134°C.

La (trifluoro-2,2,2 éthoxy)-4 aniline peut être préparée selon la méthode décrite dans le brevet US 3 920 444.

## EXEMPLE 4

A une solution refroidie à -70°C de 6,8 g de bromo-6 benzothiazolamine-2 dans 100 cm3 de tétrahydrofuranne anhydre, on ajoute, sous balayage d'argon et sous agitation, 46 cm3 d'une solution 1,6 M de n-butyllithium dans l'hexane. On laisse ensuite remonter la température vers 0°C et on ajoute une solution de 9,3 cm3 de chlorotriméthylsilane dans 10 cm3 de tétrahydrofuranne anhydre. Après retour à une température voisine de 20°C, on porte à reflux pendant 1 heure et 30 minutes. On refroidit alors vers -10°C avant d'ajouter 19 cm3 de la même solution de n-butyllithium et on laisse la température remonter vers 0°C. A la solution rouge clair obtenue, on ajoute une solution de 4,7 cm3 de chlorotriméthylsilane dans 10 cm3 de tétrahydrofuranne anhydre et on laisse la température remonter vers 20°C. On chauffe ensuite à reflux pendant 4 heures et 30 minutes. Après retour à une température voisine de 20°C, on verse le mélange réactionnel dans 100 cm3 d'eau en maintenant la température au dessous de 25°C et on alcalinise avec de l'ammoniaque. La phase aqueuse inférieure est extraite 4 fois avec 200 cm3 de dichlorométhane au total, les phases organiques sont réunies, séchées sur sulfate de magnésium, filtrées et évaporées à 40°C sous pression réduite (20 mm de mercure ; 2,7 kPa). L'huile orange obtenue (10,1 g) est chromatographiée 2 fois sur colonne de silice en éluant avec un mélange de cyclohexane et d'acétate d'éthyle (40-60 en volumes). On récupère ainsi 0,55 g d'un solide blanc qu'on triture avec 10 cm3 d'éther de pétrole (40-65°C) pour obtenir après essorage et séchage sous pression réduite (2 heures à 50°C sous 1 mm de mercure ; 0,13 kPa) 0,45 g de triméthylsilyl-6 benzothiazolamine-2 fondant à 140°C.

La bromo-6 benzothiazolamine-2 peut être préparée selon la méthode décrite dans le BEILSTEIN 27, 184.

EXEMPLE 5

On opère comme à l'exemple 1, à partir de 3,85 g de trifluorométhylthio-4 aniline, de 7 g de thiocyanate de potassium et de 1 cm3 de brome dans 30 cm3 d'acide acétique. Le produit brut marron clair est repris par 250 cm3 d'acide acétique à 80°C et la solution obtenue est traitée avec 0,4 g de noir décolorant et filtrée ; le filtrat est refroidi vers 10°C, dilué avec 150 cm3 d'eau et alcalinisé avec 400 cm3 d'ammoniaque à 28 %. Le précipité obtenu est essoré, séché à l'air et recristallisé dans un mélange de 250 cm3 de cyclohexane et 30 cm3 d'oxyde d'isopropyle. On obtient 2,9 g de trifluorométhylthio-6 benzothiazolamine-2 fondant à 155°C.

La trifluorométhylthio-4 aniline peut être préparée selon la méthode décrite dans le brevet US 2 436 100.

EXEMPLE 6

On opère comme à l'exemple 1, à partir de 0,65 g de trifluorométhoxy-4 méthyl-2 aniline, de 1,3 g de thiocyanate de potassium et de 0,35 cm3 de brome dans 12 cm3 d'acide acétique. Après purification sur colonne de silice (200 g, granulométrie : 0,063-0,200 mm) en éluant avec le mélange cyclohexane-acétate d'éthyle (50-50 en volumes), le solide blanc obtenu (0,65 g) est trituré dans 20 cm3 de cyclohexane, essoré et séché à 60 °C sous pression réduite (1 mm de mercure ; 0,13 kPa) pour donner 0,6 g de méthyl-4 trifluorométhoxy-6 benzothiazolamine-2 fondant à 162°C.

La trifluorométhoxy-4 méthyl-2 aniline peut être préparée selon la méthode décrite dans le brevet DE 3 195 926.

EXEMPLE 7

On chauffe à reflux pendant 2 heures 7,2 g de trifluorométhoxy-6 nitro-5 benzothiazolamine-2, 25 cm3 d'éthanol, 25 cm3 d'eau, 8,7 g de fer en poudre et 1,1 cm3 d'acide chlorhydrique concentré (d=1,19). Après retour à une température voisine de 20°C, on alcalinise avec 10 cm3 d'ammoniaque à 28 % et on extrait 4 fois avec 350 cm3 d'acétate d'éthyle au total. La solution organique est évaporée à 50°C sous pression réduite (20 mm de mercure ; 2,7 kPa). Le produit obtenu (6,4 g) est purifié par chromatographie sur colonne de silice (800 g, granulométrie : 0,063-0,200 mm) en éluant avec un mélange acétate d'éthyle-cyclohexane (90-10 en volumes) et recristallisé dans 320 cm3 de toluène. On obtient 4 g de trifluorométhoxy-6 benzothiazolediamine-2,5 fondant à 175°C.

La trifluorométhoxy-6 nitro-5 benzothiazolamine-2 peut être préparée de la façon suivante : à 11,7 g de trifluorométhoxy-6 benzothiazolamine-2 refroidie à -1°C, on ajoute, goutte à goutte, sous agitation mécanique, un mélange sulfonitrique refroidi vers 5°C préparé à partir de 20 cm3 d'acide sulfurique concentré (d=1,83) et de 10 cm3 d'acide nitrique concentré (d=1,42). Pendant l'addition (25 minutes) la température du mélange réactionnel est maintenue au dessous de 5°C et à la fin de l'addition l'agitation est poursuivie pendant 30 minutes entre 0°C et 2°C. On verse ensuite le produit de la réaction sur un mélange d'eau et de glace (150 cm3) et on alcalinise avec 75 cm3 d'ammoniaque à 28 %. On essore le précipité jaune obtenu qui est un mélange de trifluorométhoxy-6 nitro-5 benzothiazolamine-2 et de trifluorométhoxy-6 nitro-4 benzothiazolamine-2. Après chromatographie sur colonne de silice (1 kg, granulométrie : 0,063-0,200 mm) en éluant avec un mélange cyclohexane-acétate d'éthyle (60-40 en volumes), on obtient 9,45 g de trifluorométhoxy-6 nitro-5 benzothiazolamine-2 fondant à 260°C et 0,9 g de trifluorométhoxy-6 nitro-4 benzothiazolamine-2 fondant au-dessus de 260°C [Rf = 0,28 ; chromatographie sur couche mince de gel de silice, solvant : cyclohexane-acétate d'éthyle (50-50 en volumes)].

La trifluorométhoxy-6 benzothiazolamine-2 peut être préparée selon la méthode décrite par L.M. YAGUPOLSKII et coll., Zh. Obshch. Khim. 33, 2301 (1963).

EXEMPLE 8

On opère comme à l'exemple 7, à partir de trifluorométhoxy-6 nitro-4 benzothiazolamine-2, de fer en poudre, d'acide chlorhydrique concentré et d'éthanol à 50 % d'eau (vol./vol.). Après purification sur colonne de silice avec comme éluant un mélange de cyclohexane et d'acétate d'éthyle (10-90 en volumes) on récupère un solide blanc (1,5 g) que l'on recristallise dans 130 cm3 de toluène pour obtenir 1 g de trifluorométhoxy-6

benzothiazolediamine-2,4 fondant à 206°C.

EXEMPLE 9

On opère comme à l'exemple 1, à partir de 10 g de (tétrafluoro-1,1,2,2 éthoxy)-4 aniline, de 18,5 g de thiocyanate de potassium, de 2,4 cm3 de brome et de 80 cm3 d'acide acétique. Après purification sur colonne de silice (1 kg, granulométrie : 0,063-0,200 mm) en éluant avec un mélange de cyclohexane et d'acétate d'éthyle (50-50 en volumes) et recristallisation dans 22 cm3 de toluène, on obtient 2 g de (tétrafluoro-1,1,2,2 éthoxy)-6 benzothiazolamine-2 fondant à 161°C.

La (tétrafluoro-1,1,2,2 éthoxy)-4 aniline peut être préparée selon la méthode décrite par W.A. SHEPPARD, J. Org. Chem 29, 1 (1964).

EXEMPLE 10

A une solution de 2,1 g de (trifluoro-2,2,2 éthyl)-4 aniline dans 25 cm3 d'acide acétique, on ajoute 2,3g de thiocyanate de potassium et on agite pendant 10 minutes à une température voisine de 20°C. A la solution ainsi obtenue, on verse goutte à goutte, en 35 minutes, une solution de 0,6 cm3 de brome dans 30 cm3 d'acide acétique à une température comprise entre 20°C et 35°C. On agite ensuite pendant 16 heures à une température voisine de 20°C. Le mélange réactionnel est versé sur un mélange d'eau et de glace (150 cm3), alcalinisé avec 35 cm3 d'ammoniaque à 28 % et extrait 3 fois avec 170 cm3 d'acétate d'éthyle au total. Après décantation, la solution organique est lavée à l'eau distillée jusqu'à pH8, séchée sur sulfate de magnésium, filtrée et évaporée à 50°C sous pression réduite (20mm de mercure ; 2,7 kPa). Après purification sur une première colonne de silice en éluant avec le mélange cyclohexaneacétate d'éthyle (50-50 en volumes), on obtient un solide beige (1,7 g) fondant à 175°C, qui est chromatographié sur silice en utilisant cette fois un mélange de dichlorométhane et d'acétate d'éthyle (50-50 en volumes). On récupère ainsi 0,8 g de (trifluoro-2,2,2 éthyl)-6 benzothiazolamine-2 fondant à 186°C.

La (trifluoro-2,2,2 éthyl)-4 aniline peut être préparée de la façon suivante: à une solution de 3,8 g de (trifluoro-2,2,2 éthyl)-4 nitrobenzène dans 20 cm3 d'éthanol, on ajoute 0,17 g de charbon palladié à 10 % de palladium et on verse goutte à goutte, en 20 minutes, sous agitation, une solution de 1,8 cm3 d'hydrate d'hydrazine dans 10 cm3 d'éthanol ; on chauffe ensuite le mélange à reflux pendant 15 minutes et on laisse la température revenir vers 20°C. On filtre le catalyseur, concentre au demi le filtrat sous pression réduite (20 mm de mercure ; 2,7 kPa), ajoute 30 cm3 d'eau et extrait avec 200 cm3 d'acétate d'éthyle au total. La solution organique est séchée sur sulfate de magnésium, filtrée, évaporée sous pression réduite (20 mm de mercure ; 2,7 kPa) et le résidu d'évaporation (2,7 g) est purifié par chromatographie sur colonne de silice avec comme éluant un mélange de cyclohexane et d'acétate d'éthyle (70-30 en volumes). On obtient 2,3 g de (trifluoro-2,2,2 éthyl)-4 aniline sous forme d'huile jaune utilisée directement dans l'étape de cyclisation.

Le (trifluoro-2,2,2 éthyl)-4 nitrobenzène peut être préparé selon les méthodes décrites par S.A. FUQUA et coll., J. Org. Chem., 30, 1027 (1965), L.M. YAGUPOLSKII et coll., Synthesis, (11), 932 (1980), I. KUMADAKI et coll., J. Org. Chem., 53, 3637 (1988).

EXEMPLE 11

On opère comme à l'exemple 1, à partir de 14,6 g de pentafluoroéthyl-4 aniline, de 14 g de thiocyanate de potassium et de 3,6 cm3 de brome dans 150 cm3 d'acide acétique. Après purification sur colonne de silice avec comme éluant un mélange de cyclohexane et d'acétate d'éthyle (50-50 en volumes), on obtient un solide jaune (17 g) que l'on transforme en chlorhydrate par action d'acide chlorhydrique en solution dans l'éther éthylique. Le précipité obtenu (16 g) est recristallisé dans un mélange de 100 cm3 d'acétone et 60 cm3 d'éthanol. On obtient 3,8 g de chlorhydrate de pentafluoroéthyl-6 benzothiazolamine-2 fondant à 191°C.

La pentafluoroéthyl-4 aniline peut être préparée selon la méthode décrite dans le brevet DE 2 606 982.

EXEMPLE 12

A une solution de 2,65 g de (pentafluoro-2,2,3,3,3 propoxy)-4 aniline dans 25 cm3 d'acide acétique, on ajoute 4,27 g de thiocyanate de potassium et on agite pendant 10 minutes à une température voisine de 20°C. A la solution ainsi obtenue, on verse goutte à goutte, en 35 minutes, une solution de 0,56 cm3 de brome dans 5 cm3 d'acide acétique à une température comprise entre 22°C et 35°C, on agite ensuite pendant 16 heures à une température voisine de 20°C. Le mélange réactionnel est versé sur un mélange d'eau et de glace (150 cm3), alcalinisé avec 35 cm3 d'ammoniaque à 28 % et extrait 3 fois avec 170 cm3 d'acétate d'éthyle au total.

Après décantation, la solution organique est lavée à l'eau distillée jusqu'à pH 8, séchée sur sulfate de magnésium, filtrée et évaporée à 50°C sous pression réduite (20 mm de merure ; 2,7 kPa). Le produit obtenu (3,2 g) est purifié par chromatographie sur colonne de silice (250 g, granulométrie : 0,063-0,200 mm) avec comme éluant un mélange de cyclohexane et d'acétate d'éthyle (50-50 en volumes) et recristallisé dans 15 cm3 de toluène. On obtient 1,27 g de (pentafluoro-2,2,3,3,3 propoxy)-6 benzothiazolamine-2 fondant à 147°C.

La (pentafluoro-2,2,3,3,3 propoxy)-4 aniline peut être préparée selon la méthode décrite dans le brevet EP 205 821.

EXEMPLE 13

On opère comme à l'exemple 1, à partir de 1,5 g de benzyl-2 trifluorométhoxy-4 aniline, 2,1 g de thiocyanate de potassium dissous dans 15 cm3 d'acide acétique et de 0,88 g (0,28 cm3) de brome. L'agitation est maintenue, pendant 12 heures, à cette température. Le mélange est évaporé à sec à 80°C sous pression réduite (20 mm de mercure ; 2,7 KPa). Le résidu obtenu est repris par 10 cm3 d'eau et le pH est amené à 9-10 par de la soude concentrée (10N). Après extraction par deux fois 100 cm3 d'acétate d'éthyle, lavage des phases organiques réunies par deux fois 50 cm3 d'eau, séchage sur du sulfate de magnésium anhydre et évaporation à sec à 40°C sous pression réduite (20 mm de mercure ; 2,7 KPa), on isole 1,3 g de benzyl-4 trifluorométhoxy-6 benzothiazolamine-2 fondant à 175°C.

La benzyl-2 trifluorométhoxy-4 aniline peut être préparée de la façon suivante : 1,7 g de N-(benzyl-2 trifluorométhoxy-4 phényl) tert-butylcarboxamide, 22 cm3 d'acide chlorhydrique concentré (12N) dissous dans 35 cm3 d'eau sont chauffés au reflux pendant 12 heures. Après neutralisation par de la soude concentrée (10N) jusqu'à pH 11 et extraction par deux fois 50 cm3 d'acétate d'éthyle et évaporation à sec à 40°C sous pression réduite (20 mm de mercure 2,7 KPa), on isole 1,5 g de benzyl-2 trifluorométhoxy-4 aniline se présentant sous forme d'une huile orangée.

Le N-(benzyl-2 trifluorométhoxy-4 phényl) tert-butylcarboxamide peut être préparé de la façon suivante : à un mélange de 1,1 g d'hydrure de sodium à 50 % en dispersion dans l'huile de vaseline dissous dans 24 cm3 de sulfure de carbone, on ajoute, goutte à goutte 11 g de N-(hydroxyphénylméthyl-2 trifluorométhoxy-4 phényl) tert-butylcarboxamide dissous dans 96 cm3 de sulfure de carbone à une température comprise entre 30 et 34°C. La solution est laissée 1 heure à 25°C et on y ajoute goutte à goutte 42,6 g (19 cm3) d'iodure de méthyle. On laisse le tout sous agitation à 25°C pendant une nuit. Après addition de 150 cm3 d'une solution saturée de chlorure d'ammonium, extraction par 100 cm3 de dichlorométhane, séchage sur du sulfate de magnésium anhydre et concentration sous pression réduite (20 mm de mercure ; 2,7 KPa), on isole 3,4 g d'une huile jaune. Cette huile est directement dissoute dans 25 cm3 de toluène avec 0,07 g d'azobisisobutyronitrile et le tout est porté à 80°C. On y additionne, goutte à goutte et sous azote, 8 cm3 d'hydrure de tributylétain. La solution est laissée à 45 minutes à 85°C. Après évaporation à sec à 30°C sous pression réduite (20 mm de mercure ; 2,7 KPa), le résidu obtenu est purifié par flash-chromatographie sur colonne de silice, sous courant d'azote, à moyenne pression (0,5-1,5 bar) avec un mélange de cyclohexane et d'acétate d'éthyle (95-5 en volumes) comme éluant. On isole 1,7 g N-(benzyl-2 trifluorométhoxy-4 phényl) tert-butylcarboxamide fondant à 88°C.

Le N-(hydroxyphénylméthyl-2 trifluorométhoxy-4 phényl) tert-butylcarboxamide peut être préparé de la façon suivante : à 15,7 g de N-(trifluorométhoxy-4) tert-butylcarboxamide dissous dans 75 cm3 de tétrahydrofuranne, à 0°C, sous agitation, on ajoute, en 30 minutes 75 cm3 de n-butyl-lithium (1,6M dans l'hexane). Le mélange est agité à une température de 0°C pendant 3 heures. On additionne ensuite 7 g (6,7 cm3) de benzaldéhyde et on laisse le tout 12 heures à 25°C. Après addition de 200 cm3 d'eau, extraction par deux fois 100 cm3 d'acétate d'éthyle, séchage sur du sulfate de magnésium et concentration sous pression réduite (20 mm de mercure ; 2, KPa), on isole 14,1 g de N-(hydroxyphénylméthyl-2 trifluorométhoxy-4 phényl) tert-butylcarboxamide fondant à 140°C.

Les médicaments selon l'invention sont constitués par au moins un composé de formule (I) ou un sel d'un tel composé à l'état pur ou sous forme d'une composition dans laquelle il est associé à tout autre produit pharmaceutiquement compatible, pouvant être inerte ou physiologiquement actif. Ces médicaments peuvent être employés par voie orale, parentérale, rectale ou topique.

Comme compositions solides pour administration orale, peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels

que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propyléneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthyléneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300 mg par jour par voie orale pour un adulte avec des doses unitaires allant de 10 à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

Exemple A

On prépare, selon la technique habituelle, des gélules dosées à 50 mg de produit actif ayant la composition suivante :

| | |
|---|---|
| - pentafluoroéthoxy-6 benzothiazolamine-2 | 50 mg |
| - cellulose | 18 mg |
| - lactose | 55 mg |
| - silice colloïdale | 1 mg |
| - carboxyméthylamidon sodique | 10 mg |
| - talc | 10 mg |
| - stéarate de magnésium | 1 mg |

Exemple B

On prépare, selon la technique habituelle, des comprimés dosés à 50 mg de produit actif ayant la composition suivante :

- tertbutyl-6 benzothiazolamine-2 .................... 50 mg
- lactose .......................................... 104 mg
- cellulose ......................................... 40 mg
- polyvidone ........................................ 10 mg
- carboxyméthylamidon sodique ........................ 22 mg
- talc .............................................. 10 mg
- stéarate de magnésium .............................. 2 mg
- silice colloïdale .................................. 2 mg
- mélange d'hydroxyméthylcellulose, glycérine, oxyde de titane (72-3,5-24,5) .................. q. s. p. 1 comprimé pelliculé terminé à 245 mg

Exemple C

On prépare une solution injectable contenant 10 mg de produit actif ayant la composition suivante :

- triméthylsilyl-6 benzothiazolamine-2 ................ 10 mg
- acide benzoïque .................................... 80 mg
- alcool benzylique .................................. 0,06 cm3
- benzoate de sodium ................................. 80 mg
- éthanol à 95 % ..................................... 0,4 cm3
- hydroxyde de sodium ................................ 24 mg
- propylèneglycol .................................... 1,6 cm3
- eau ......................................... q. s. p. 4 cm3

## Revendications

### Revendications pour les etats contractants suivants : AT, BE, CH, DE, FR, GB, IT, LI, LU, NL, SE

1. Médicaments contenant en tant que principe actif au moins un composé de formule :

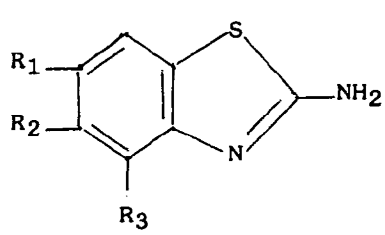

(I)

dans laquelle

– soit $R_1$ représente un radical polyfluoroalcoxy, trifluoro-2,2,2 éthyle, pentafluoroéthyle, tertbutyle, triméthylsilyle ou trifluorométhylthio et $R_2$ et $R_3$ représentent un atome d'hydrogène,

– soit $R_1$ représente un radical polyfluoroalcoxy, $R_2$ représente un atome d'hydrogène et $R_3$ représente un radical alkyle, amino ou phènylalkyle,

– soit $R_1$ représente un radical polyfluoroalcoxy, $R_2$ représente un radical amino et $R_3$ représente un atome

d'hydrogène à l'exception de la trifluorométhoxy-6 benzothiazolamine-2 et étant entendu que les radicaux ou les portions alkyle et alcoxy contiennent 1 à 4 atomes de carbone en chaîne droite ou ramifiée ou un sel d'un tel composé avec un acide minéral ou organique.

2. Médicaments selon la revendication 1 pour lesquels le radical polyfluoroalcoxy est un radical pentafluoroéthoxy, trifluoro-2,2,2 éthoxy, tétrafluoro-1,1,2,2 éthoxy, trifluorométhoxy ou pentafluoro-2,2,3,3,3 propoxy.

3. Médicament selon les revendications 1 ou 2 pour le traitement des affections où la glutamate est impliqué.

4. Composés de formule :

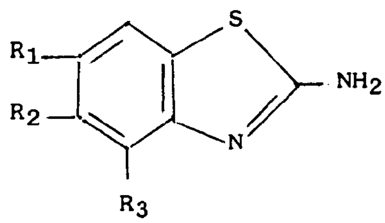

(I)

dans laquelle

– soit $R_1$ représente un radical polyfluoroalcoxy, trifluoro-2,2,2 éthyle, pentafluoroéthyle, tertbutyle ou triméthylsilyle et $R_2$ et $R_3$ représentent un atome d'hydrogène,

– soit $R_1$ représente un radical polyfluoroalcoxy, $R_2$ représente un atome d'hydrogéne et $R_3$ représente un radical alkyle amino ou phénylalkyle,

– soit $R_1$ représente un radical polyfluoroalcoxy, $R_2$ représente un radical amino et $R_3$ représente un atome d'hydrogène à l'exception de la trifluorométhoxy-6 benzothiazolamine-2 et étant entendu que les radicaux ou les portions alkyle et alcoxy contiennent 1 à 4 atomes de carbone en chaîne droite ou ramifiée ou un sel d'un tel composé avec un acide minéral ou organique.

5. Composés selon la revendication 4 pour lesquels le radical polyfluoroalcoxy est un radical pentafluoroéthoxy, trifluoro-2,2,2 éthoxy, tétrafluoro-1,1,2,2 éthoxy, trifluorométhoxy ou pentafluoro-2,2,3,3,3 propoxy.

6. Procédé de préparation des composés de formule (I) selon la revendication 4 pour lesquels

– soit $R_1$ représente un radical polyfluoroalcoxy, trifluoro-2,2,2 éthyle, pentafluoroéthyle ou tertbutyle et $R_2$ et $R_3$ représentent un atome d'hydrogène,

– soit $R_1$ représente un radical polyfluoroalcoxy, $R_2$ représente un atome d'hydrogène et $R_3$ représente un radical alkyle, ou phénylalkyle caractérisé en ce que l'on fait réagir un thiocyanate de métal alcalin et du brome sur une amine de formule :

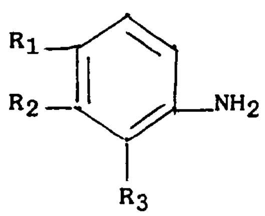

(II)

dans laquelle $R_1$, $R_2$ et $R_3$ ont les mêmes significations que précédemment, isole le produit et le transforme éventuellement en sel avec un acide minéral ou organique.

7. Procédé de préparation du composé de formule (I) selon la revendication 4 pour lequel $R_1$ représente un radical triméthylsilyle et $R_2$ et $R_3$ représentent un atome d'hydrogène caractérisé en ce que l'on fait réagir du chlorotriméthylsilane sur le dérivé lithié de la N,N-bistriméthylsilyl benzothiazolamine-2 obtenu par action de butyllithium et de chlorotriméthylsilane sur la bromo-6 benzothiazolamine puis hydrolyse, isole le produit et le transforme éventuellement en sel avec un acide minéral ou organique.

8. Procédé de préparation du composé de formule (I) selon la revendication 4 pour lequel $R_1$ représente un radical polyfluoroalcoxy et

– soit $R_2$ représente un radical amino et $R_3$ représente un atome d'hydrogène,

– soit $R_2$ représente un atome d'hydrogène et $R_3$ représente un radical amino,

caractérisé en ce que l'on réduit un composé de formule :

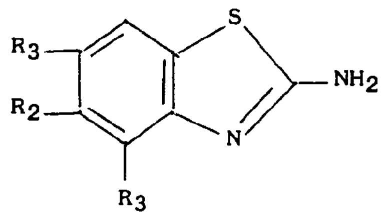

(III)

dans laquelle $R_1$ représente un radical polyfluoroalcoxy et soit $R_2$ représente un radical nitro et $R_3$ représente un atome d'hydrogène, soit $R_2$ représente un atome d'hydrogène et $R_3$ représente un radical nitro, isole le produit et le transforme éventuellement en sel avec un acide minéral ou organique.

**Revendications pour les Etats contractants suivants : ES, GR**

1. Procédé de préparation des composé de formule :

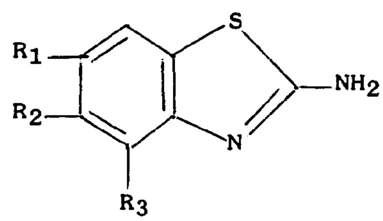

(I)

dans laquelle

– soit $R_1$ représente un radical polyfluoroalcoxy, trifluoro-2,2,2 éthyle, pentafluoroèthyle, tertbutyle ou triméthylsilyle et $R_2$ et $R_3$ représentent un atome d'hydrogène,

– soit $R_1$ représente un radical polyfluoroalcoxy, $R_2$ représente un atome d'hydrogène et $R_3$ représente un radical alkyle amino ou phénylalkyle,

– soit $R_1$ représente un radical polyfluoroalcoxy, $R_2$ représente un radical amino et $R_3$ représente un atome d'hydrogène à l'exception de la trifluorométhoxy-6 benzothiazolamine-2 et étant entendu que les radicaux ou les portions alkyle et alcoxy contiennent 1 à 4 atomes de carbone en chaîne droite ou ramifiée ou un sel d'un tel composé avec un acide minéral ou organique, caractérisé en ce que :

A - pour la préparation des composés de formule (I) pour lesquels

– soit $R_1$ représente un radical polyfluoroalcoxy trifluoro-2,2,2 éthyle, pentafluoroéthyle ou tertbutyle et $R_2$ et $R_3$ représentent un atome d'hydrogène,

– soit $R_1$ représente un radical polyfluoroalcoxy, $R_2$ représente un atome d'hydrogène et $R_3$ représente un radical alkyle ou phénylalkyle on fait réagir un thiocyanate de métal alcalin et du brome sur une amine de formule :

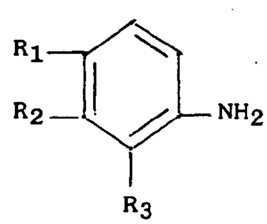

(II)

dans laquelle $R_1$, $R_2$ et $R_3$ ont les mêmes significations que précédemment, isole le produit et le transforme éventuellement en sel avec un acide minéral ou organique.

B - pour la préparation du composé de formule (I) pour lequel $R_1$ représente un radical triméthylsilyle et $R_2$ et $R_3$ représentent un atome d'hydrogène on fait réagir du chlorotriméthylsilane sur le dérivé lithié en 6 de la N,N-bistriméthylsilyl benzothiazolamine-2 puis hydrolyse, isole le produit et le transforme éventuellement en sel avec un acide minéral ou organique.

C - pour la préparation des composés de formule (I) pour lesquels $R_1$ représente un radical polyfluoroalcoxy et soit $R_2$ représente un radical amino et $R_3$ représente un atome d'hydrogène, soit $R_2$ repré-

sente un atome d'hydrogène, soit $R_2$ représente un atome d'hydrogène et $R_3$ représente un radical amino on réduit un dérivé de formule :

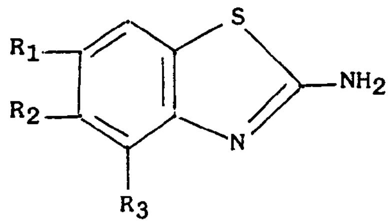

(III)

dans laquelle $R_1$ représente un radical polyfluoroalcoxy et soit $R_2$ représente un radical nitro et $R_3$ un atome d'hydrogène, soit $R_2$ représente un atome d'hydrogène et $R_3$ représente un radical nitro, isole le produit et le transforme éventuellement en sel d'addition avec un acide minéral ou organique.

2. Procédé selon la revendication 1 pour la préparation de composés de formule (I) pour lesquels le radical polyfluoroalcoxy est un radical pentafluoroéthoxy, trifluoro-2,2,2 éthoxy, tétrafluorofluoro-1,1,2,2 éthoxy, trifluorométhoxy ou pentafluoro-2,2,3,3,3 propoxy.

## Patentansprüche

### Patentansprüche für folgende Vertragsstaaten : AT, BE, CH, DE, FR, GB, IT, LI, LU, NL, SE

1. Medikamente, die als aktiven Bestandteil zumindest eine Verbindung der Formel:

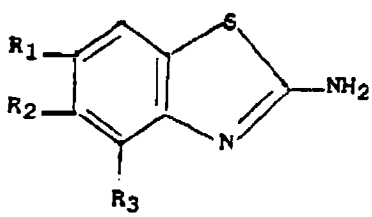

(I)

in welcher

– entweder $R_1$ einen Polyfluoralkoxy-, 2,2,2-Trifluoräthyl-, Pentafluoräthyl-, tert.Butyl-, Trimethylsilyl- oder Trifluormethylthiorest darstellt und $R_2$ und $R_3$ ein Wasserstoffatom darstellen,

– oder $R_1$ einen Polyfluoralkoxyrest darstellt, $R_2$ ein Wasserstoffatom darstellt und $R_3$ einen Alkyl-, Amino- oder Phenylalkylrest darstellt,

– oder $R_1$ einen Polyfluoralkoxyrest darstellt, $R_2$ einen Aminorest darstellt und $R_3$ ein Wasserstoffatom darstellt, mit Ausnahme von 6-Trifluormethoxy-2-benzothiazolamin, wobei die Alkyl- und Alkoxyreste oder -teile selbstverständlich 1 bis 4 Kohlenstoffatome in gerader oder verzweigter Kette enthalten, oder ein Salz einer solchen Verbindung mit einer anorganischen oder organischen Säure enthalten.

2. Medikamente nach Anspruch 1, für welche der Polyfluoralkoxyrest ein Pentafluoräthoxy-, 2,2,2-Trifluoräthoxy-, 1,1,2,2-Tetrafluoräthoxy-, Trifluormethoxy- oder 2,2,3,3,3-Pentafluorpropoxyrest ist.

3. Medikament nach den Ansprüchen 1 oder 2 zur Behandlung von Störungen, an denen Glutamat beteiligt ist.

4. Verbindungen der Formel:

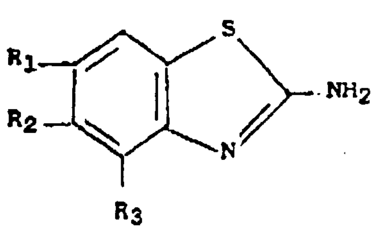

(I)

in welcher

– entweder $R_1$ einen Polyfluoralkoxy-, 2,2,2-Trifluoräthyl-, Pentafluoräthyl-, tert.Butyl- oder Trimethylsilylrest darstellt und $R_2$ und $R_3$ ein Wasserstoffatom darstellen,

– oder $R_1$ einen Polyfluoralkoxyrest darstellt, $R_2$ ein Wasserstoffatom darstellt und $R_3$ einen Alkyl-, Amino- oder Phenylalkylrest darstellt,

– oder $R_1$ einen Polyfluoralkoxyrest darstellt, $R_2$ einen Aminorest darstellt und $R_3$ ein Wasserstoffatom darstellt, mit Ausnahme von 6-Trifluormethoxy-2-benzothiazolamin, wobei die Alkyl- und Alkoxyreste oder -teile selbstverständlich 1 bis 4 Kohlenstoffatome in gerader oder verzweigter Kette enthalten, oder ein Salz einer solchen Verbindung mit einer anorganischen oder organischen Säure.

5. Verbindungen nach Anspruch 4, dadurch gekennzeichnet, für welche der Polyfluoralkoxyrest ein Pentafluoräthoxy-, 2,2,2-Triftuoräthoxy-, 1,1,2,2-Tetrafluoräthoxy-, Triftuormethoxy- oder 2,2,3,3,3-Pentafluorpropoxyrest ist.

6. Verfahren zur Herstellung von Verbindungen der Formel (I) nach Anspruch 4, für welche

– entweder $R_1$ einen Polyfluoralkoxy-, 2,2,2-Trifluoräthyl-, Pentafluoräthyl- oder tert.Butylrest darstellt und $R_2$ und $R_3$ ein Wasserstoffatom darstellen,

– oder $R_1$ einen Polyfluoralkoxyrest darstellt, $R_2$ ein Wasserstoffatom darstellt und $R_3$ einen Alkylrest oder Phenylalkylrest darstellt, dadurch gekennzeichnet, daß man ein Alkalimetallthiocyanat und Brom mit einem Amin der Formel:

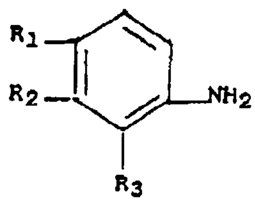

(II)

reagieren läßt, in welcher $R_1$, $R_2$ und $R_3$ die gleichen Bedeutungen wie zuvor haben, das Produkt isoliert und es gegebenenfalls in ein Salz mit einer anorganischen oder organischen Säure umwandelt.

7. Verfahren zur Herstellung der Verbindung der Formel (I) nach Anspruch 4, worin $R_1$ einen Trimethylsilylrest darstellt und $R_2$ und $R_3$ ein Wasserstoffatom darstellen, dadurch gekennzeichnet, daß man Chlortrimethylsilan mit der Lithiumverbindung von N,N-Bistrimethylsilyl-2-benzothiazolamin, erhalten durch Umsetzung von Butyllithium und Chlortrimethylsilan mit 6-Brombenzothiazolamin, reagieren läßt, dann hydrolysiert, das Produkt isoliert und es gegebenenfalls in ein Salz mit einer anorganischen oder organischen Säure umwandelt.

8. Verfahren zur Herstellung der Verbindung der Formel (I) nach Anspruch 4, worin $R_1$ einen Polyfluoralkoxyrest darstellt und

– entweder $R_2$ einen Aminorest darstellt und $R_3$ ein Wasserstoffatom darstellt,

– oder $R_2$ ein Wasserstoffatom darstellt und $R_3$ einen Aminorest darstellt,

dadurch gekennzeichnet, daß man eine Verbindung der Formel:

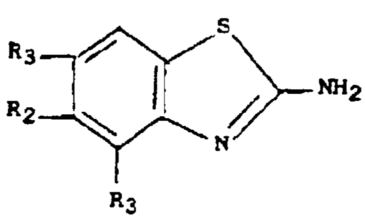

(III)

in welcher $R_1$ einen Polyfluoralkoxyrest darstellt und entweder $R_2$ einen Nitrorest darstellt und $R_3$ ein Wasserstoffatom darstellt, oder $R_2$ ein Wasserstoffatom darstellt und $R_3$ einen Nitrorest darstellt, reduziert, das Produkt isoliert und es gegebenenfalts in ein Salz mit einer anorganischen oder organischen Säure umwandelt.

**Patentansprüche für folgende Vertragsstaaten : ES, GR**

1. Verfahren zur Herstellung von Verbindungen der Formel:

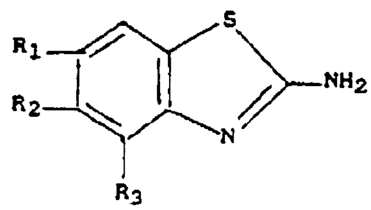

(I)

in welcher

– entweder $R_1$ einen Polyfluoralkoxy-, 2,2,2-Trifluoräthyl-, Pentafluoräthyl-, tert.Butyl- oder Trimethylsilylrest darstellt und $R_2$ und $R_3$ ein Wasserstoffatom darstellen,

– oder $R_1$ einen Polyfluoralkoxyrest darstellt, $R_2$ ein Wasserstoffatom darstetlt und $R_3$ einen Alkyl-, Amino- oder Phenylalkylrest darstellt,

– oder $R_1$ einen Polyfluoralkoxyrest darstellt, $R_2$ einen Aminorest darstellt und $R_3$ ein Wasserstoffatom darstellt, mit Ausnahme von 6-Trifluormethoxy-2-benzothiazolamin, wobei die Alkyl- und Alkoxyreste oder -teile selbstverständlich 1 bis 4 Kohlenstoffatome in gerader oder verzweigter Kette enthalten, oder eines Salzes einer solchen Verbindung mit einer anorganischen oder organischen Säure, dadurch gekennzeichnet, daß man

A - zur Herstellung von Verbindungen der Formel (I), worin

– entweder $R_1$ einen Polyfluoralkoxy-, 2,2,2-Trifluoräthyl-, Pentafluoräthyl-, oder tert.Butylrest darstellt und $R_2$ und $R_3$ ein Wasserstoffatom darstellen,

– oder $R_1$ einen Polyfluoralkoxyrest darstellt, $R_2$ ein Wasserstoffatom darstellt und $R_3$ einen Alkyl- oder Phenylalkylrest darstellt, ein Alkalimetallthiocyanat und Brom mit einem Amin der Formel

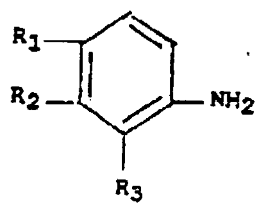

(II)

in welcher $R_1$, $R_2$ und $R_3$ die gleichen Bedeutungen wie zuvor haben, reagieren läßt, die Verbindung isoliert und sie gegebenenfalls in ein Satz mit einer anorganischen oder organischen Säure umwandelt,

B - zur Herstellung der Verbindung der Formel (I), in welcher $R_1$ einen Trimethylsilylrest darstellt und $R_2$ und $R_3$ ein Wasserstoffatom darstellen, Chlortrimethylsilan auf eine in 6-Stellung lithiumhaltige Verbindung von N,N-Bistrimethylsilyl-2-benzothiazolamin einwirken läßt, dann hydrolysiert, das Produkt isoliert und es gegebenenfalls in ein Satz mit einer anorganischen oder organischen Säure umwandelt,

C - zur Herstellung von Verbindungen der Formel (I), worin $R_1$ einen Polyfluoralkoxyrest darstellt und entweder $R_2$ einen Aminorest darstellt und $R_3$ Wasserstoff darstellt oder $R_2$ ein Wasserstoffatom darstellt und $R_3$ einen Aminorest darstellt, eine Verbindung der Formel:

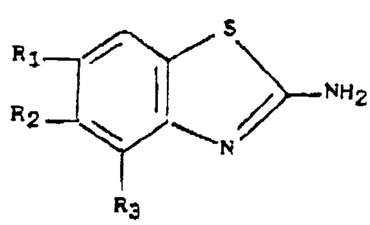

(III)

in welcher $R_1$ einen Polyfluoralkoxyrest darstellt und entweder $R_2$ einen Nitrorest und $R_3$ ein Wasserstoffatom darstellt oder $R_2$ ein Wasserstoffatom und $R_3$ einen Nitrorest darstellt, reduziert, das Produkt isoliert und es gegebenenfalls in ein Additionssalz mit einer anorganischen oder organischen Säure überführt.

2. Verfahren nach Anpruch 1 zur Herstellung von verbindungen der Formel (I), worin der Polyfluoralkoxyrest ein Pentafluoräthoxy-, 2,2,2-Trifluoräthoxy-, 1,1,2,2-Tetrafluoräthoxy-, Trifluormethoxy- oder 2,2,3,3,3-Pentafluorpropoxyrest ist.

## Claims

### Claims for the following Contracting States : AT, BE, CH, DE, FR, GB, IT, LI, LU, NL, SE

1. Medicinal products containing, as active principle, at least one compound of formula:

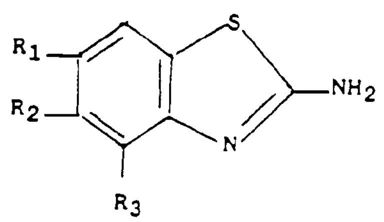

(I)

in which

– either $R_1$ denotes a polyfluoroalkoxy, 2,2,2-trifluoroethyl, pentafluoroethyl, tert-butyl, trimethylsilyl or trifluoromethylthio radical and $R_2$ and $R_3$ denote a hydrogen atom,

– or $R_1$ denotes a polyfluoroalkoxy radical, $R_2$ denotes a hydrogen atom and $R_3$ denotes an alkyl, amino or phenylalkyl radical,

– or $R_1$ denotes a polyfluoroalkoxy radical, $R_2$ denotes an amino radical and $R_3$ denotes a hydrogen atom, with the exception of 6-trifluoromethoxy-2-benzothiazolamine and on the understanding that the alkyl and alkoxy radicals or portions contain 1 to 4 carbon atoms in a straight or branched chain, or a salt of such a compound with an inorganic or organic acid.

2. Medicinal products according to Claim 1, for which the polyfluoroalkoxy radical is a pentafluoroethoxy, 2,2,2-trifluoroethoxy, 1,1,2,2-tetrafluoroethoxy, trifluoromethoxy or 2,2,3,3,3-pentafluoropropoxy radical.

3. Medicinal product according to Claim 1 or 2, for the treatment of conditions in which glutamate is implicated.

4. Compounds of formula:

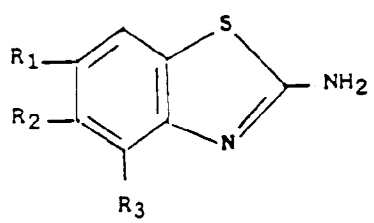

(I)

in which

– either $R_1$ denotes a polyfluoroalkoxy, 2,2,2-trifluoroethyl, pentafluoroethyl, tert-butyl or trimethylsilyl radical and $R_2$ and $R_3$ denote a hydrogen atom,

– or $R_1$ denotes a polyfluoroalkoxy radical, $R_2$ denotes a hydrogen atom and $R_3$ denotes an alkyl, amino or phenylalkyl radical,

– or $R_1$ denotes a polyfluoroalkoxy radical, $R_2$ denotes an amino radical and $R_3$ denotes a hydrogen atom, with the exception of 6-trifluoromethoxy-2-benzothiazolamine and on the understanding that the alkyl and alkoxy radicals or portions contain 1 to 4 carbon atoms in a straight or branched chain, or a salt of such a compound with an inorganic or organic acid.

5. Compounds according to Claim 4, for which the polyfluoroalkoxy radical is a pentafluoroethoxy, 2,2,2-trifluoroethoxy, 1,1,2,2-tetrafluoroethoxy, trifluoromethoxy or 2,2,3,3,3-pentafluoropropoxy radical.

6. Process for preparing the compounds of formula (I) according to Claim 4 for which

– either $R_1$ denotes a polyfluoroalkoxy, 2,2,2-trifluoroethyl, pentafluoroethyl or tert-butyl radical and $R_2$ and $R_3$ denote a hydrogen atom,

– or $R_1$ denotes a polyfluoroalkoxy radical, $R_2$ denotes a hydrogen atom and $R_3$ denotes an alkyl or phenylalkyl radical, characterised in that an alkali metal thiocyanate and bromine are reacted with an amine of formula:

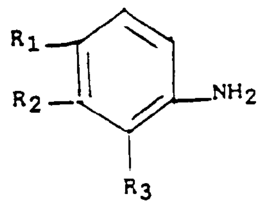

(II)

in which $R_1$, $R_2$ and $R_3$ have the same meanings as above, and the product is isolated and optionally converted to a salt with an inorganic or organic acid.

7. Process for preparing the compound of formula (I) according to Claim 4 for which $R_1$ denotes a trimethylsilyl radical and $R_2$ and $R_3$ denote a hydrogen atom, characterised in that chlorotrimethylsilane is reacted with the lithium derivative of N,N-bis(trimethylsilyl)-2-benzothiazolamine obtained by the action of butyllithium and chlorotrimethylsilane on 6-bromobenzothiazolamine, the resulting compound is then hydrolysed, and the product is isolated and optionally converted to a salt with an inorganic or organic acid.

8. Process for preparing the compound of formula (I) according to Claim 4 for which $R_1$ denotes a polyfluoroalkoxy radical and

– either $R_2$ denotes an amino radical and $R_3$ denotes a hydrogen atom,

– or $R_2$ denotes a hydrogen atom and $R_3$ denotes an amino radical,

characterised in that a compound of formula:

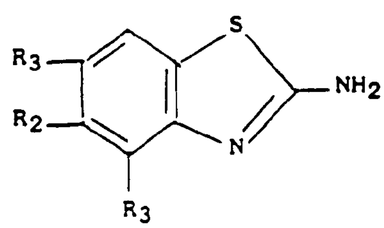

(III)

in which $R_1$ denotes a polyfluoroalkoxy radical, and either $R_2$ denotes a nitro radical and $R_3$ denotes a hydrogen atom or $R_2$ denotes a hydrogen atom and $R_3$ denotes a nitro radical, is reduced, and the product is isolated and optionally converted to a salt with an inorganic or organic acid.

**Claims for the following Contracting States: ES, GR**

1. Process for preparing the compounds of formula:

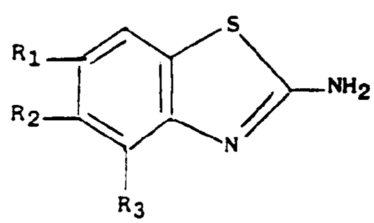

(I)

in which

– either $R_1$ denotes a polyfluoroalkoxy, 2,2,2-trifluoroethyl, pentafluoroethyl, tert-butyl or trimethylsilyl radical and $R_2$ and $R_3$ denote a hydrogen atom,

– or $R_1$ denotes a polyfluoroalkoxy radical, $R_2$ denotes a hydrogen atom and $R_3$ denotes an alkyl, amino or phenylalkyl radical,

– or $R_1$ denotes a polyfluoroalkoxy radical, $R_2$ denotes an amino radical and $R_3$ denotes a hydrogen atom, with the exception of 6-trifluoromethoxy-2-benzothiazolamine and on the understanding that the alkyl and alkoxy radicals or portions contain 1 to 4 carbon atoms in a straight or branched chain, or a salt of such a compound with an inorganic or organic acid, characterised in that:

A - for the preparation of the compounds of formula (I) for which

– either $R_1$ denotes a polyfluoroalkoxy, 2,2,2-tri-fluoroethyl, pentafluoroethyl or tert-butyl radical and

$R_2$ and $R_3$ denote a hydrogen atom,
– or $R_1$ denotes a polyfluoroalkoxy radical, $R_2$ denotes a hydrogen atom and $R_3$ denotes an alkyl or phenylalkyl radical, an alkali metal thiocyanate and bromine are reacted with an amine of formula:

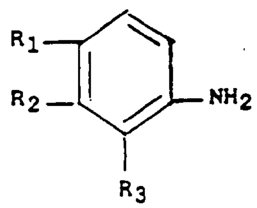

(II)

in which $R_1$, $R_2$ and $R_3$ have the same meanings as above, and the product is isolated and optionally converted to a salt with an inorganic or organic acid.

B - for the preparation of the compound of formula (I) for which $R_1$ denotes a trimethylsilyl radical and $R_2$ and $R_3$ denote a hydrogen atom, chlorotrimethylsilane is reacted with the N,N-bis(trimethylsilyl)-2-benzo-thiazolamine derivative lithiated at the 6-position, the resulting compound is then hydrolysed, and the product is isolated and optionally converted to a salt with an inorganic or organic acid.

C - for the preparation of the compounds of formula (I) for which $R_1$ denotes a polyfluoroalkoxy radical and either $R_2$ denotes an amino radical and $R_3$ denotes a hydrogen atom, or $R_2$ denotes a hydrogen atom, or $R_2$ denotes a hydrogen atom and $R_3$ denotes an amino radical, a derivative of formula:

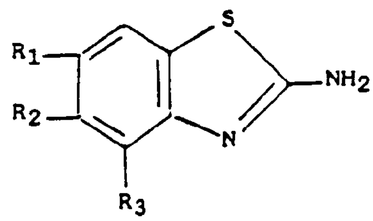

(III)

in which $R_1$ denotes a polyfluoroalkoxy radical, and either $R_2$ denotes a nitro radical and $R_3$ denotes a hydrogen atom or $R_2$ denotes a hydrogen atom and $R_3$ denotes a nitro radical, is reduced, and the product is isolated and optionally converted to an addition salt with an inorganic or organic acid.

2. Process according to Claim 1 for the preparation of compounds of formula (I) for which the polyfluoroalkoxy radical is a pentafluoroethoxy, 2,2,2-trifluoroethoxy, 1,1,2,2-tetrafluoroethoxy, trifluoromethoxy or 2,2,3,3,3-pentafluoropropoxy radical.